# EUROPEAN PATENT APPLICATION

(11) **EP 4 112 041 A1**
(43) Date of publication of application: **04.01.2023**
(21) Application number: 21382580.5
(22) Date of filing: 30.06.2021
(51) Int. Cl.: A61K 9/00, A61K 9/06, A61K 9/08, A61K 31/196, A61K 47/34, A61P 19/02, A61P 21/00, A61P 29/00

(54) **MICELLAR SOLUTION COMPRISING DICLOFENAC**

(71) Applicant: GSK Consumer Healthcare SARL, 1197 Prangins (CH)
(72) Inventor: Alonso, Maria José, 15782 Santiago de Compostela (ES); Teijeiro, Desirée, 15782 Santiago de Compostela (ES); Catanzano, Ovidio, 15782 Santiago de Compostela (ES); Berrecoso Cuña, Germán, 15782 Santiago de Compostela (ES); Ronsoni Zancán, Lali, 15782 Santiago de Compostela (ES)
(74) Representative: Haleon Patent Department

(57) **Abstract**

There are provided novel topical or transdermal nano-formulations of diclofenac. The formulations are in the form of micellar solutions. The micellar solutions comprise diclofenac alkali metal salt, in a concentration of about 2% to about 5.5% (w/w), a solvent, a mixture of surfactants, and an aqueous continuous, outer phase. The micellar solution may optionally further comprise permeation enhancers. The micellar solutions can be gelled with a suitable gelling agent. The compositions disclosed are useful in the treatment of joint pain, osteoarthritis, muscle pain, back pain and/or inflammation.

## Description

### FIELD OF THE INVENTION

This disclosure relates to nano-formulations for the topical delivery of diclofenac. Specifically, it relates to a novel micellar solution formulation designed for about 2% to about 5.5% (w/w) diclofenac sodium. The micellar solutions provide high and/or prolonged permeation of the active through the skin.

### BACKGROUND TO THE INVENTION

Topical, transdermal products comprising diclofenac, a non-steroidal anti-inflammatory drug (NSAID) of the acetic acid class, are currently available to patients and consumers in Europe and other countries, and are widely used for their analgesic and anti-inflammatory properties, providing an advantage over oral NSAID formulations, inter alia due to reduced systemic adverse effects. However, it is very difficult to develop topical formulations that are pharmaceutically acceptable and at the same time exhibit consumer preferred attributes. Additionally, diclofenac is a particularly challenging ingredient to work with, and poses many challenges to the formulator. Improved topical compositions comprising diclofenac are needed that meet pharmaceutical, technological and consumer expectations.

### SUMMARY OF THE INVENTION

There is provided a micellar solution for topical drug delivery, comprising:
a) diclofenac alkali metal salt, in a concentration of about 2% to about 5.5% (w/w),
b) diethylene glycol monoethyl ether in a concentration of about 3% to about 7% (w/w),
c) propylene glycol esters of C₆ to C₁₂ fatty acids,
d) polyethylene glycol esters of C₁₄ to C₁₈ fatty acids,
e) polyethylene glycol esters of sorbitan C₁₆ to C₂₀ fatty acid monoester, and
f) water,
wherein c) and d) together are present in a concentration of about 10% to about 25% (w/w), and
wherein e) is present in a concentration of about 5% to about 10% (w/w).

In one embodiment, component c) has a hydrophilic lipophilic balance of about 5 to about 7.

In one embodiment, component d) has a hydrophilic lipophilic balance of about 15 to about 17.

In one embodiment, component e) has a hydrophilic lipophilic balance of about 14 to about 16.

In one embodiment, component c) is present in a concentration of about 5% to about 15% (w/w).

In one embodiment, component c) is propylene glycol monocaprylate.

In one embodiment, component d) is present in a concentration of about 10% to about 20% (w/w).

In one embodiment, component d) is a mixture of polyethylene glycol mono- and diesters of 12-hydroxystearic acid, comprising about 20% to about 40% (w/w) free polyethylene glycol.

In one embodiment, component e) is polyethylene glycol sorbitan monooleate.

In one embodiment, the micellar solution is transparent.

In one embodiment, the micellar solution has an average micelle size, determined by dynamic light scattering, in the range of about 5nm to about 10nm.

In one embodiment, the micelles of the micellar solution form spontaneously.

In one embodiment, the micellar solution comprises:
a) about 4% to about 5% (w/w) diclofenac sodium,
b) about 5% (w/w) diethylene glycol monoethyl ether,
c) about 5% (w/w) Capryol 90,
d) about 10% (w/w) Kolliphor HS15,
e) about 5% (w/w) Tween 80, and
f) water.

In one embodiment, the micellar solution is for use in the treatment of joint pain, osteoarthritis, muscle pain, back pain and/or inflammation.

In one embodiment, the micellar solution is provided in a container, the container comprising an applicator for direct application onto the skin.

There is also provided a gel for topical drug delivery, comprising:
a) the micellar solution as disclosed herein, in a concentration of about 40% to about 80% (w/w),
b) a gelling agent, selected from xanthan gum, hydroxyethyl cellulose, and mixtures thereof.

In one embodiment, the gel further comprises:
c) a permeation enhancer, selected from C₁₂ to C₂₀-fatty alcohols, esters of C₈ to C₁₀-saturated fatty acids with C₁₂ to C₁₈-fatty alcohols, N-methyl-2-pyrrolidone and N-dodecyl caprolactam.

In one embodiment, the gel further comprises:
d) a C₂ to C₅ poly alcohol,
e) isopropanol, in a concentration of about 8% to about 12% (w/w),
f) sorbitan monolaurate, and
g) polysorbate 20.

In one embodiment, component b) is xanthan gum, present in a concentration of about 1.5 to about 2.5% (w/w), and component c) is oleyl alcohol, present in a concentration of about 0.75% (w/w).

### DESCRIPTION OF DRAWINGS/FIGURES

FIG. 1: Schematic drawing of a micellar solution according to this disclosure.
FIG. 2: Illustrative photographs of selected micellar solutions of this disclosure.
FIG. 3: Illustrative photographs of selected gel formulations based on micellar solutions, containing a final concentration of about 2.15 % (w/w) of diclofenac sodium.

### DETAILED DESCRIPTION OF THE INVENTION

To facilitate extended therapeutic efficacy, an important objective of the inventors has been to provide a composition which facilitates improved diclofenac penetration through the skin, into regions of interest for anti-inflammatory and anti-nociception effects. The skin, however, is a natural barrier, providing hydrophobic qualities to minimise percutaneous absorption of ionised drugs, such as diclofenac.

One way to improve penetration of diclofenac is through incorporation of higher concentrations of the active pharmaceutical ingredient (API). This however entails numerous difficulties. Higher amounts of API can lead to chemical instability due to a higher or lower pH. Higher amounts of API can also cause instability due to interaction with other ingredients of the formulation. The approach of raising API concentration is especially limited for diclofenac, due to the very limited solubility and high cristallisation tendency of diclofenac. Furthermore, higher API concentrations render products more expensive, and enlarge their environmental footprint. Inter alia for environmental reasons, it is desirable to achieve the highest possible penetration with the least concentration of active. Thereby, API usage could be reduced without compromising efficacy. Furthermore, exposure of the environment with "unused" active could be reduced.

Another way to achieve higher penetration of diclofenac is through incorporation of (higher) concentrations of permeation enhancer(s) in a formulation. However, permeation enhancer(s) can also cause skin irritation, and can, in some concentrations, even have a penetration retarding effect. Penetration enhancers can also lead to instability of multiphase topical systems. Additionally, product characteristics like rheology or appearance can be negatively impacted by inclusion of permeation enhancers. It would be preferred to provide a product that achieves high penetration of diclofenac even without the need to incorporate a permeation enhancer. But ideally, penetration enhancers could nevertheless be successfully incorporated into the formulation to further enhance penetration of the API, without a negative impact on stability, product attributes and safety. Thereby, a product range with different strengths or potencies could be created, based on the base formulation.

Several challenges lie in the manufacture of topical products. Many methods of manufacture are cumbersome, expensive and waste (water) intensive, e.g. because they require numerous vessels. Another problem specific to multiphase systems is the energy impact of usual emulsification methods. Using (high) shear homogenization, especially for prolonged periods of time, is energy intensive. This leads to higher production cost and can also negatively impact stability because of higher temperatures and oxygen introduction. Therefore, there is a need for a product that can be manufactured in an economic and environmentally friendly method of manufacture, without high energy impact.

An additional challenge is to provide a stable composition suitable for commercial distribution and sale. The minimum stability profile requires overcoming challenges including the resistance to phase separation in multiphase systems, creaming, precipitation and flocculation.

Another challenge is to provide a topical product with pleasant attributes. Currently, consumers prefer non greasy, light products. Also preferred are clear, transparent or translucent topical products. Ideally the products should have pleasing spread characteristics, and dry quickly without leaving residue on the skin. The texture of the product needs to be pleasant to consumers. A certain viscosity is required to provide good spreading. However, incorporation of gelling agents into multiphase systems often leads to considerable problems such as phase separation, clouding of the composition, discoloration or flaking after application on the skin. The rheology of the product should be such that exact doses can be measured without the need of special equipment. Cooling properties are often preferred, however incorporation of excipients with a cooling effect is also limited due to various formulation challenges.

Consequently, there remains a need for a topical, transdermal diclofenac formulation that addresses one or more of the described challenges above.

### DEFINITIONS

As used herein, a micellar solution is a composition with a hydrophilic, polar continuous phase. Dispersed in the continuous phase are aggregates of amphiphilic molecules, wherein the aggregates are in the form of micelles. In the hydrophilic continuous phase, hydrophilic or lipophobic moieties of the amphiphilic molecules are oriented towards the continuous phase, i.e. towards a surface of the micelles, whereas hydrophobic or lipophilic moieties are oriented towards a core of the micelles. The micelles have an average size below 100nm, usually determined by dynamic light scattering (DLS) technique. The micelles might therefore also be referred to as nano-micelles. The micelles in the micellar solution have the potential to accommodate hydrophobic or lipophilic components or ingredients in the core, yielding nano-emulsions.

Unless indicated otherwise, concentrations are given in %(w/w).

The term "about" in relation to a numerical value x means, for example, x±10%, x±5%, x±4%, x±3%, x±2%, x±1%.

### PHARMACEUTICAL COMPOSITIONS

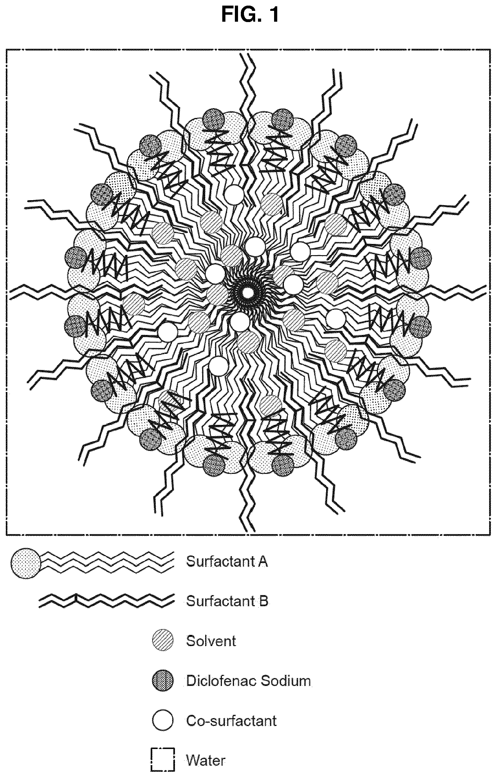

This disclosure provides pharmaceutical compositions for topical drug delivery which are micellar solutions with an aqueous outer phase. Micelles, formed by a mixture of surfactants, are distributed in the outer aqueous phase. The micelles comprise the active pharmaceutical ingredient (API) (referred to as component a) herein), surfactants (referred to as first, second and third surfactants, or components c), d) and e) herein), and a solvent (referred to as component b) herein), as can be seen in the schematic drawing of FIG. 1. In FIG. 1, a micelle is shown comprising surfactants A and B, which are surfactants with a hydrophilic lipophilic balance (HLB) value of above 15. Surfactants A and B form the micelle, with their hydrophobic moieties oriented towards a core of the micelle and their hydrophilic moieties oriented towards the continuous phase, which is water (referred to as component f) herein). In the compositions of the invention, the functionality of surfactants A and B is provided by a surfactant referred to as second surfactant, or component d) herein, and a surfactant referred to as third surfactant, or component e) herein. A co-surfactant, having an HLB value of below 8, can aid spontaneous self-assembly of nano-micelles (referred to as first surfactant, or component c) herein). A solvent can be present in the core of the micelle. In the compositions of this disclosure, the solvent is referred to as component b). Without being bound to this theory, it is believed that diclofenac cations, having amphiphilic properties, are located in a margin of the micelle, with their hydrophilic carboxylate head group on the micelle surface, oriented towards the aqueous outer phase, and their hydrophobic body of the molecule oriented towards the core of the micelle.

In one embodiment, the micellar solution has a negative zeta potential of about - 50mV to about -5mV, about -45mV to about -5mV, or about -35mV to about -5mV. In one embodiment, the micelles have a narrow size variation which can be expressed in a polydispersion index (PDI) of below about 0.4, below about 0.3 or below about 0.2. In one embodiment, the micelles have a PDI of about 0.1 to 0.1 to about 0.4. It may be observed that for some compositions, initial PDI (directly after manufacture) can be higher, but declines upon storage to a final, lower PDI in the stated range.

Preferably, the compositions have a pH above about 6. More preferred the pH is in the range of about 6 to about 8.

This disclosure also provides pharmaceutical gel compositions for topical drug delivery, comprising a micellar solution as disclosed herein, and a gelling agent. The aqueous outer phase of the micellar solution is gelled by the addition of specific gelling agents that are compatible with the micelles.

The compositions disclosed herein comprise diclofenac alkali metal salt as the active pharmaceutical ingredient (API). In one embodiment, the diclofenac alkali metal salt is diclofenac sodium, diclofenac potassium or a mixture thereof. In a preferred embodiment, the diclofenac alkali metal salt is diclofenac sodium.

Preferably, diclofenac is the only API in the compositions.

### Micellar solutions

The micellar solution preferably comprises diclofenac alkali metal salt in a concentration of about 2% to about 5.5% (w/w) (component **a**)). Preferably, the micellar solution comprises about 2.5% to about 5.5% (w/w) diclofenac alkali metal salt. In another preferred embodiment, the micellar solution comprises about 4% to about 5% (w/w) diclofenac alkali metal salt. In another preferred embodiment, the micellar solution comprises about 4% to about 5% (w/w) diclofenac sodium.

The micellar solution of this disclosure comprises a solvent (component **b**)). The solvent is a non-volatile solvent. Preferably, the solvent is a solvent of the glycol, or glycol ether or glycol ether ester family. Preferably, the solvent has a boiling point of above about 150°C at normal pressure. Preferably, the solvent has an octanol/water partition coefficient expressed as log P_{ow} of about -0.5 to about 1 at 20°C. In one preferred embodiment, the solvent is diethylene glycol monoethyl ether.

In one embodiment, the solvent is present in a concentration of about 3% to about 7% (w/w). In a preferred embodiment, the solvent is present in a concentration of about 5% (w/w).

The micellar solution of this disclosure comprises a combination of surfactants (components **c)**, **d)** and **e)**). The surfactants can be present in a concentration above their critical micelle concentration. In one embodiment, the micellar solution comprises the micellar solution comprises two surfactants (namely component c) and d)). In a preferred embodiment, the micellar solution comprises at least three surfactants. In an alternative embodiment the micellar solution comprises three surfactants.

The combination of surfactants provides for self-assembling nano-micelles. In one embodiment, the micellar solution comprises a first surfactant with a hydrophilic lipophilic balance (HLB) value of about 5-7 and a second, and optionally further surfactants with an HLB value of about 14 to 17. In one embodiment, the micellar solution comprises a first surfactant with an HLB value of about 5-7, a second surfactant with an HLB value of about 15-17 and a third surfactant with an HLB value balance of about 14 to 16. Optionally, there may be more than one first, second or third surfactant, which have an HLB value within these ranges.

In one embodiment, the micellar solution comprises a surfactant which is a propylene glycol ester of C₆ to C₁₂ fatty acids. An example is Capryol 90. In one embodiment, the micellar solution comprises a surfactant which is a polyethylene glycol ester of C₁₄ to C₁₈ fatty acids. An example is Macrogol-15-Hydroxystearate. In one embodiment, the micellar solution comprises a surfactant which is a polyethylene glycol ester of sorbitan C₁₆ to C₂₀ fatty acid monoester. One example is Tween 80. In one embodiment, the micellar solution comprises a surfactant combination which consists of a propylene glycol ester of C₆ to C₁₂ fatty acids, a polyethylene glycol ester of C₁₄ to C₁₈ fatty acids and a polyethylene glycol ester of sorbitan C₁₆ to C₂₀ fatty acid monoester. In one embodiment, the first surfactant is propylene glycol monocaprylate. In one embodiment, the second surfactant is a mixture of polyethylene glycol mono- and diesters of 12-hydroxystearic acid, or Macrogol-15-Hydroxystearate, or a mixture of polyethylene glycol mono- and diesters of 12-hydroxystearic acid comprising about 20% to about 40% (w/w) free polyethylene glycol. In one embodiment, the third surfactant is polyethylene glycol sorbitan monooleate.

In one embodiment, the first surfactant and second surfactant are present in a cumulative concentration of about 10% to about 25% (w/w). In one embodiment, the first surfactant is present in a concentration of about 5% to about 15% (w/w). In one embodiment, the second surfactant is present in a concentration of about 10% to about 20% (w/w). In one embodiment, the third surfactant is present in a concentration of about 5% to about 10% (w/w).

The outer phase of the micellar solutions is water. It may be pharma grade water or purified water or any other water suitable for pharmaceutical compositions. In one embodiment, the micellar solution comprises at least about 50%, at least about 60%, at least about 70%, or at least about 75% (w/w) water.

It has surprisingly been found by the present inventors that the micellar solutions of the present disclosure can be diluted, without harming the properties of the micelles, with water, and optionally additional ingredients. The micellar solutions may be diluted using about 70% to about 80% micellar solution. Alternatively, the micellar solution can be diluted using as few as about 40% of micellar solution.

Preferably, the micellar solution is translucent or transparent. In one preferred embodiment, the micellar solution is transparent. Translucent or transparent compositions are often preferred by patients and can lead to a better compliance. Micellar solutions with an average micelle size, determined by dynamic light scattering, of about 10nm to about 50nm, can be regarded as translucent. Micellar solutions with an average micelle size, determined by dynamic light scattering, of about 5nm to about 10nm, can be regarded as transparent. Average micelle size is the average size of the micelles present in the micellar solution, determined by dynamic light scattering.

In one embodiment, micellar solution has an average micelle size, determined by dynamic light scattering, in the range of about 2nm to about 50nm. In one embodiment, the micellar solution has an average micelle size, determined by dynamic light scattering, in the range of about 6nm to about 20nm. In one embodiment, the micellar solution has an average micelle size, determined by dynamic light scattering, in the range of about 5nm to about 15nm. In one embodiment, the micellar solution has an average micelle size, determined by dynamic light scattering, in the range of about 4nm to about 12nm. In one embodiment, the micellar solution has an average micelle size, determined by dynamic light scattering, in the range of 5nm to 10nm.

Preferably, the micellar solution is self-assembling. In other words, amphiphilic molecules in the described composition aggregate into a micelle structure upon mixture of the ingredients. This process involves complex interfacial hydrodynamic phenomena and depends on the system composition properties. Only very specific pharmaceutical excipient combinations and concentrations lead to efficient self-assembly into micellar solutions. Preferably, the micellar solution spontaneously forms nano-micelles with an average micelle size (measured with dynamic light scattering) of the micelles of about 4nm to about 12nm. Self-assembling micelles are preferred because they are more stable, less susceptible of phase separation, creaming, flocculation and the like. They are also preferred because these do not require high energy manufacturing methods like micro fluidization, high pressure homogenization, ultrasonic treatment or the like.

Optionally, permeation enhancers may be incorporated into the micellar solutions. The pharmaceutical formulator may be aware of many permeation enhancers; however it is a challenge to successfully incorporate permeation enhancers into multi-phase systems, let alone nano-micelle-systems like the micellar solutions of the present disclosure. Incorporation of permeation enhancers may cause instability of the multi-phase system, leading to phase separation or coalescence of the nano-micelles to larger micelles, or even creaming, phase separation or similar. The present inventors have surprisingly found that permeation enhancers selected from the group of C₁₂-C₂₀-fatty alcohol, esters of C₈-C₁₀-saturated fatty acids with C₁₂-C₁₈-fatty alcohols, N-methyl-2-pyrrolidone, N-dodecyl caprolactam, and propylene glycol, or any combination thereof, can successfully be incorporated into the disclosed micellar solutions.

In one embodiment, the permeation enhancer is oleyl alcohol. Preferably, oleyl alcohol is present in a concentration of about 0.25% to about 1.5% (w/w). More preferred, oleyl alcohol is present in a concentration of about 0.5% to about 1% (w/w). Most preferred, oleyl alcohol is present in a concentration of about 0.75% (w/w).

In one embodiment, the permeation enhancer is cocoyl caprylo caprate. In another embodiment, the permeation enhancer is N-methyl-2-pyrrolidone. In again another embodiment, the permeation enhancer is N-dodecyl caprolactam. Cocoyl caprylo caprate can be present in concentrations of about 0.5% to about 1% (w/w). N-methyl-2-pyrrolidone can be present in concentrations of up to about 5% (w/w), for example about 2.5% to about 5% (w/w). N-dodecyl caprolactam can be present in concentrations of about 0.5% to about 5% (w/w). Propylene glycol can be present in concentrations of about 2% to about 15% (w/w) or about 5% to about 10% (w/w).

Optionally, the micellar solutions may further comprise ethanol or isopropanol. Preferred is isopropanol. Ethanol or isopropanol may be present in a concentration of about 5% to about 20% (w/w). In one embodiment, isopropanol is present in a concentration of about 8% to about 12% (w/w). These agents reduce the time that is needed to upon rubbing the micellar solution into the skin until the skin feels dry (drying time). It was surprisingly found that said alcohols can successfully be incorporated into the micellar solutions in the above concentration range.

The micellar solution may comprise further excipients, such as perfumes, chelating agents, preservatives, antioxidants or the like.

In one embodiment, the micellar solution comprises:
a) diclofenac alkali metal salt, in a concentration of about 2% to about 5.5% (w/w),
b) glycol, or glycol ether, or glycol ether ester, in a concentration of about 3% to about 7% (w/w),
c) a first surfactant with an HLB value of about 5-7, in a concentration of about 5% to about 15% (w/w),
d) a second surfactant with an HLB value of about 15-17, in a concentration of about 10% to about 20% (w/w),
e) a third surfactant with an HLB value of about 14-16, in a concentration of about 5% to about 10% (w/w),
f) water, in a concentration of about 40% to about 80% (w/w).

In one embodiment, the micellar solution comprises:
a) diclofenac alkali metal salt, in a concentration of about 4% to about 5% (w/w),
b) glycol ether, in a concentration of about 3% to about 7% (w/w),
c) a first surfactant with an HLB value of about 5-7, selected from propylene glycol esters of C₆ to C₁₂ fatty acids, in a concentration of about 5% to about 15% (w/w),
d) a second surfactant with an HLB value of about 15-17, selected from polyethylene glycol esters of C₁₄ to C₁₈ fatty acids, in a concentration of about 10% to about 20% (w/w),
e) a third surfactant with an HLB value of about 14-16, selected from polyethylene glycol esters of sorbitan C₁₆ to C₂₀ fatty acid monoester, in a concentration of about 5% to about 10% (w/w),
f) water, in a concentration of about 40% to about 80% (w/w),
   wherein c) and d) together are present in a concentration of about 10% to about 25% (w/w).

In one embodiment, the micellar solution comprises:
a) in a concentration of about 3% to about 4% (w/w) diclofenac sodium,
b) about 3% to about 4% % (w/w) diethylene glycol monoethyl ether and about 4% to about 6% propylene glycol,
c) about 3% to about 4%% (w/w) Capryol 90,
d) about 7% to about 8% (w/w) Kolliphor HS15,
e) about 3% to about 4% (w/w) Tween 80 and about 1% to about 2% (w/w) Tween 20,
   optionally, ethanol, isopropanol, or a mixture thereof, in a concentration of about 5% to about 15% (w/w),
   optionally, permeation enhancers in a concentration of about 0.5% to about 5% (w/w), and
f) water, q.s. to 100% (w/w).

In one embodiment, the micellar solution consists essentially of:
a) about 4% to about 5% (w/w) diclofenac sodium,
b) about 5% (w/w) diethylene glycol monoethyl ether,
c) about 5% (w/w) Capryol 90,
d) about 10% (w/w) Kolliphor HS15,
e) about 5% (w/w) Tween 80,
f) water q.s. to 100% (w/w).

### Gel compositions

This disclosure also provides pharmaceutical gel compositions for topical drug delivery, comprising a micellar solution as disclosed herein, and a gelling agent. Gels are preferable for topical administration because they provide convenient and exact dosing, even without a dosing device, and generally are easy to handle with few dripping. Gels are also preferred because they provide a pleasant feeling during application and are easy to rub in. Many gelling agents are known to the pharmaceutical formulator. However, the gelling agent and the network the gelling agent builds in a given formulation upon gelling can disrupt multi-phase systems like emulsions and micelles, and therefore it is difficult to provide stable, gelled emulsions and micelles. This is even more complicated for nano-systems like micellar solutions of the present disclosure. The gelling agent may not disrupt nano-micelles, and may not cause them to coalesce which would ultimately lead to emulsion breaking over time. In other cases, the micellar solution can hamper the gelling capacity of the gelling agent, whereby it is not possible to increase the viscosity of the micellar solutions with that gelling agent.

The present inventors have surprisingly found that the micellar solutions of the present disclosure can be formulated into gels, whilst maintaining the nano-micellar structure of the micellar solution. Concentrations values for ingredients, given in relation to the gel compositions of the present disclosure, are concentrations (w/w) of the ingredients in the final gel formulation.

The gels of the present disclosure comprise the micellar solution as described herein as a first component (component **a**)), and a gelling agent as a second component (component **b**)).

Preferably, the micellar solution is present in a concentration of about 30% to about 80% (w/w). More preferred, the micellar solution is present in a concentration of about 40% to about 60% (w/w).

The gelling agent is selected from xanthan gum, hydroxyethyl cellulose, and a mixture thereof. Preferably, the gelling agent is xanthan gum.

Preferably, xanthan gum is present in a concentration of about 1% to about 3% (w/w). More preferably, xanthan gum is present in a concentration of about 1.2% to about 2% (w/w). Preferably, hydroxyethyl cellulose is present in a concentration of about 1.2% to about 2% (w/w).

Like the micellar solutions of the present disclosure, the gel compositions may further comprise a permeation enhancer (component **c**)), selected from C₁₂ to C₂₀-fatty alcohol, esters of C₈ to C₁₀-saturated fatty acids with C₁₂ to C₁₈-fatty alcohols, N-methyl-2-pyrrolidone and N-dodecyl caprolactam. As discussed above, incorporation of a permeation enhancer in nano-micellar compositions is a major challenge. The permeation enhancers compatible with the micellar solutions of the present disclosure may also be incorporated into the gel compositions. Surprisingly, neither nano-micelle structure, nor gel building capacity are negatively impacted by these permeation enhancers.

As discussed above for the micellar solution, the gel compositions may equally optionally comprise ethanol or isopropanol. The present inventors have surprisingly found that ethanol and isopropanol, in a concentration of about 8% to about 12% (w/w), may be incorporated into the gel compositions.

In one embodiment, the gel comprises:
a) diclofenac alkali metal salt in a concentration of about 1% to about 4.5% (w/w),
b) glycol, or glycol ether, or glycol ether ester in a concentration of about 1.5% to about 5.5% (w/w),
c) a first surfactant with an HLB value of about 5-7 in a concentration of about 2% to about 12% (w/w),
d) a second surfactant with an HLB value of about 15-17, in a concentration of about 4% to about 16% (w/w),
e) a third surfactant with an HLB value of about 14-16 in a concentration of about 2% to about 10% (w/w),
   a gelling agent, selected from xanthan gum, hydroxyethyl cellulose, and any combination thereof in a concentration of about 0.5% to about 2.5% (w/w), water, in a concentration of about 40% to about 80% (w/w).

In one embodiment, the gel comprises:
a) about 2% to about 4% (w/w) diclofenac sodium,
b) about 1.5% to about 3% % (w/w) diethylene glycol monoethyl ether and 4% to 6% propylene glycol,
c) about 2% to about 3% (w/w) Capryol 90,
d) about 4% to about 6% (w/w) Kolliphor HS15,
e) about 2% to about 4% (w/w) Tween 80 and about 0.5% to about 1% (w/w) Tween 20,
   about 0.5% to about 2.5% (w/w of a gelling agent, selected from xanthan gum, hydroxyethyl cellulose, and any combination thereof,
   about 5% to about 15% (w/w) of isopropanol or ethanol or a combination thereof,
   about 2% to about 7% (w/w),propylene glycol, and
   water, q.s. ad 100% (w/w).

In one embodiment, the gel consists essentially of:
Diclofenac sodium in a concentration of about 2% to about 3% (w/w),
Capryol 90, in a concentration of about 2% to about 3% (w/w),
Kolliphor HS15, in a concentration of about 4% to about 5% (w/w),
Tween 80, in a concentration of about 2% to about 3% (w/w),
Diethylene glycol monoethyl ether, in a concentration of about 2% to about 3% (w/w),
Isopropanol, in a concentration of about 8% to about 12% (w/w),
Propylene glycol, in a concentration of about 4% to about 6% (w/w),
Oleyl alcohol, in a concentration of about 0.5% to about 1.5% (w/w),
N-Methyl-2-pyrrolidone, in a concentration of about 4% to about 6% (w/w),
Span 80, in a concentration of about 0.5% to about 1.5% (w/w),
Tween 20, in a concentration of about 0.5% to about 1.5% (w/w),
Hydroxyethyl cellulose, in a concentration of about 1.5% to about 2.5% (w/w) and
Purified water, q.s. ad 100%.

### METHODS OF MANUFACTURE

### Micellar solution

The micellar solutions may be manufactured by mixing components a) b), c), d) and e) in a vessel under stirring at about 40 °C at about 700 rotations per minute (rpm). Stirring is continued for about 30 minutes until a) is completely dissolved, and the mixtures forms a homogenous, first phase. Optionally, additional, hydrophilic excipients may be solubilised in component f) in a separate vessel, to form an aqueous phase. Alternatively pure water is used as the aqueous phase. The aqueous phase is added over the first phase under continuous stirring for about 30 minutes at about 700rpm at about 40°C. The vessel should be covered with a lid during the procedure.

The following procedure can be used to incorporate additional ingredients into the micellar solution, for example ethanol, isopropanol, permeation enhancers, or other optional ingredients:
The desired amount (usually 40% to 80% (w/w) of the total final composition) of micellar solution, prepared as described above, is mixed in a vessel with the additional ingredient(s), under continuous stirring at about 700rpm for about 30 minutes at about 40°C. The vessel should be covered with a lid during the procedure.

### Gel compositions

The gels of the present disclosure, which comprise the micellar solution and a gelling agent, can be manufactured as follows:
(1) a desired amount of micellar solution can be diluted with water to a desired API concentration,
(2) gelling agent is dispersed into the mixture of the selected amount of micellar solution and water,
(3) optionally, permeation enhancers can be added, mixed for about 30 seconds at high shear (about 2000rpm), and then stirred for about 30 minutes at room temperature.

### METHODS OF TREATMENT

The micellar solutions may be administered to mammals suffering from joint pain, osteoarthritis, muscle pain, back pain and/or inflammation. Preferably, the micellar solutions are for the treatment of humans. The micellar solutions are useful in the treatment of joint pain, osteoarthritis, muscle pain, back pain and/or inflammation. They can be applied onto the skin covering the concerned body part. They may be rubbed in until the skin feels dry. They may be applied 2-4 times per day. Alternatively, they may be applied once per day. Typical doses of the micellar solutions comprise 0.09mg diclofenac equivalent/ cm² (2-4 times per day application) or 0.21mg diclofenac equivalent/ cm² (twice per day application). The methods of treatment can comprise administering to a subject in need thereof a pharmaceutically effective amount of the micellar solution or gel compositions disclosed herein. The method can comprise administering the composition to skin that covers a body part suffering from or causing one or more of joint pain, osteoarthritis, muscle pain, back pain and/or inflammation.

The micellar solutions may be provided in a container comprising an applicator for direct application onto the skin. The micellar solutions may be provided in a package comprising a means for dosing. In one embodiment, the dosing means is a pump. In one embodiment, the means for dosing is a pipette.

The gel compositions of the present disclosure may be administered to patients suffering from joint pain, osteoarthritis, muscle pain, back pain and/or inflammation. The gel compositions are useful in the treatment of joint pain, osteoarthritis, muscle pain, back pain and/or inflammation. They can be applied onto the skin covering the concerned body part. They may be rubbed in until the skin feels dry. They may be applied 2-4 times per day. Alternatively, they may be applied once per day. Typical doses of the gel compositions comprise 0.09mg diclofenac equivalent/ cm² (2-4 times per day application) or 0.21mg diclofenac equivalent/ cm² (twice per day application).

The gel compositions may be provided in a container comprising an applicator for direct application onto the skin.

### EXAMPLES

### Initial Screening

In a formulation screening, 34 formulations were developed using different ratios of excipients listed in Table 1.

**Table 1. List of raw materials and concentration range for the preparation of micellar solutions.**

| Component | | Concentration [% w/w] |
|---|---|---|
| a) | Diclofenac sodium | 2.5 - 7.5 |
| b) | Diethylene glycol monoethyl ether | 5 |
| c) | Capryol 90 | 9 - 25 |
| d) | Tween 80 | 5 - 10 |
| e) | Kolliphor HS15 | 10 - 20 |
| f) | Water | q.s. ad 100 |

| | | |
|---|---|---|
| q.s.: quantum satis | | |

The components b), c), d) and e) were first mixed under stirring at 40°C to form a first phase. Diclofenac sodium was added and completely dissolved. The water, f), was added over first oily phase under continuous stirring.

The 34 formulations were stored at room temperature for one day. Only the formulations which were physically stable one day after preparation were characterized in terms of micelle size, poly dispersion index (PDI), zeta potential, physical stability upon storage at room temperature after 15 days, and pH, see the results in Table 2.

If not indicated otherwise, herein, average micelle size and polydispersity index (PDI) was determined by dynamic light scattering (DLS), and zeta potential was determined by laser Doppler electrophoresis, in a Nanosizer ZS (Malvern Instruments) at 25°C and 173° measurement angle. pH was measured with a pH-meter PB11 Sartorius, and diclofenac assay was performed with ultra performance liquid chromatography (ACQUITY UPLC, Waters).

**Table 2: Composition, characterization and 15-days stability of micellar solutions containing a 2% Diclofenac sodium, called "M-series" of the screening.**

| | Composition | | | Initial Characterization | | | | Operational stability Time: 15 days |
|---|---|---|---|---|---|---|---|---|
| USC Code | Capryol 90 | Kolliphor | Tween 80 | DicNa | Size (nm) | PDI | ZP (mV) | Appearance |
| M 11 2% Dic Na | 5 | 20 | 10 | 2.15 | 6.1 | 0.2 | -8 | Transparent |
| M 12 2% Dic Na | 10 | 20 | 10 | 2.15 | 4.5 | 0.2 | -31 | Phase Separation |
| M 13 2% Dic Na | 15 | 20 | 10 | 2.15 | 5.5 | 0.3 | -6 | Transparent |
| M 14 2% Dic Na | 20 | 20 | 10 | 2.15 | (**) | | -12 | Transparent |
| M 15 2% Dic Na | 25 | 20 | 10 | 2.15 | (**) | | -21 | Phase Separation |
| M 16 2% Dic Na | 5 | 10 | 5 | 2.15 | 6.1 | 0.1 | -19 | Transparent |
| M 17 2% Dic Na | 10 | 10 | 5 | 2.15 | 18 | 0.1 | -33 | Transparent |
| M 18 2% Dic Na | 15 | 10 | 5 | 2.15 | 11.2 | 0.4 | -12 | Transparent |
| M 19 2% Dic Na | 20 | 10 | 5 | 2.15 | 14.1 | 0.4 | -30 | Phase Separation |
| M 20 2% Dic Na | 25 | 10 | 5 | 2.15 | 128 | 0.5 | -28 | Transparent |

| | | | | | | | | |
|---|---|---|---|---|---|---|---|---|
| All formulations contain 5% of Transcutol All formulation show transparent appearance (**) oily external phase | | | | | | | | |

Only four of these formulations were physically stable with no sign of phase separation after 15 days, had an aqueous outer phase, and had a micelle size of less than 50nm, and were thus considered suitable. Among these four micellar solutions, one formulation was selected for further development and experimentation, namely:

### Increase of drug loading

It was surprisingly found that these formulations have high drug solubilisation capacity, especially for diclofenac. This enabled to markedly increase the drug loading in the micellar solutions. Six formulations based on formulation number M16 were prepared with higher concentrations of diclofenac sodium (4.3 - 7%), and subjected to a stability program (two months storage at 25°C/65%RH and 40°C/75%RH ICH conditions). Afterwards, the diclofenac content in the upper and lower half of the storage vial was measured.

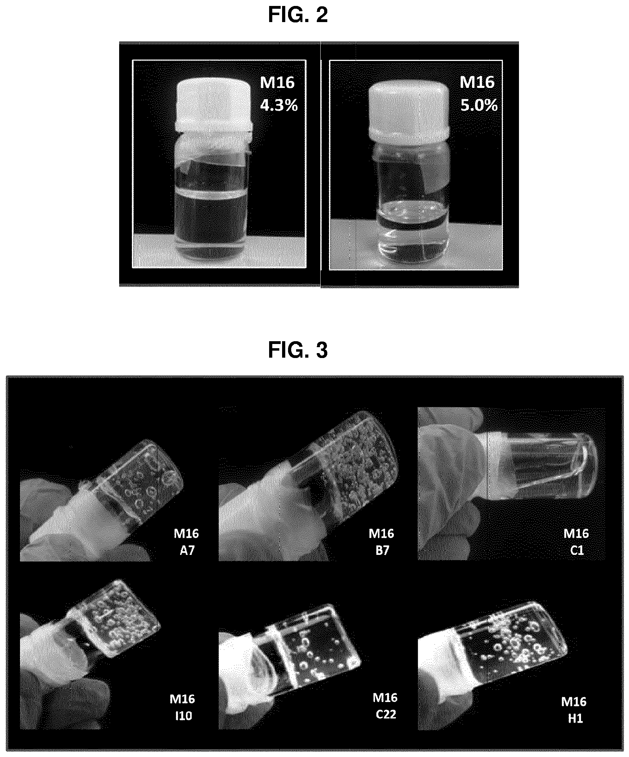

Interestingly, formulations based on M16 with up to a 5% diclofenac sodium loading (FIG. 2) were stable after 60 days at both 25°C/65%RH and 40°C/75%RH ICH conditions, and maintained the drug content within the acceptable limits (Table 3). Formulations comprising 6% (w/w) or higher showed phase separation after storage under both tested conditions for two months.

Table 3. Initial characterization and 2-month ICH stability at both 25°C and 40ºC of M16 micellar solutions containing increasing diclofenac loadings (4.3 - 7%) [Drug Up/down % means drug content compared to initial drug content in the samples from the upper (Up) and lower (down) half of the storing-vials

### Development of topical gels, based on the micellar solutions

| | | | | | ICH Stability | | | | | | | |
|---|---|---|---|---|---|---|---|---|---|---|---|---|
| | Initial Characterization | | | Time | 25°C ± 2°C/60% RH ± 5% | | | | 40°C ± 2°C/75% RH ± 5% | | | |
| Sample name | Size (nm) | pH | Drug (%) | (months) | Size (nm) | pH | Drug up (%) | Drug Down (%) | Size (nm) | pH | Drug up (%) | Drug down (%) |
| M16 4.3%DicNa | 7.02 | 7.2 | 102 | 1 | 12.1 | 7.2 | 86* | 97 | 10.5 | 7.1 | 93 | 95 |
| | | | | 2 | 21.7 | 7 | 99 | 110 | 20.5 | 7 | 112 | 110 |
| M16 5.0% DicNa | 19.3 | 7.1 | 100 | 1 | 17.4 | 7.1 | 79* | 92 | 21.8 | 7.1 | 97 | 97 |
| | | | | 2 | 24.2 | 7.1 | 95 | 94 | 19.3 | 7 | 101 | 100 |
| M16 5.5%DicNa | 52.2 | 7.4 | 105 | 1 | 27.5 | 7.3 | 87* | 102 | 29 | 7.2 | 95 | 88 |
| | | | | 2 | 36.1 | 7.1 | 95 | 98 | 55 | 7.1 | 109 | 105 |
| M16 6.0%DicNa | 95 | 7.3 | 97 | 1 | 106 | 7.3 | 96 | 92 | 186 | 7.2 | 94 | 107 |
| | | | | 2 | Phase Separation | | | | Phase Separation | | | |
| M16 6.5%DicNa | 98 | 7.5 | 107 | 1 | 110 | 7.4 | 97 | 92 | 209 | 7.4 | 86 | 96 |
| | | | | 2 | Phase Separation | | | | Phase Separation | | | |
| M16 7.0%DicNa | 7.8 | 7.7 | 116 | 1 | 52 | 7.5 | 90 | 88 | 222 | 7.4 | 113 | 111 |
| | | | | 2 | Phase Separation | | | | Phase Separation | | | |

| | | | | | | | | | | | | |
|---|---|---|---|---|---|---|---|---|---|---|---|---|
| (*)Probable analytical/sampling error | | | | | | | | | | | | |

### Development of topical gels, based on the micellar solutions

It was tried to viscosify the micellar solution comprising 4.3% (w/w) diclofenac sodium of the previous experiments with usual gelling agents. Among the different gelling agents evaluated, it was possible to increase the viscosity up to obtaining a semisolid form with Xantural 95, a xanthan gum in a concentration of 1.2% to 2.0% (w/w) and Natrosol 250M, a hydroxyethyl cellulose, in a concentration of 1.2% to 2.0% (m/v). All alternative viscosifying agents employed caused instability (phase separation/precipitation) or gelling failure.

### Screening for incorporation of permeation enhancers

Taking advantage of the high diclofenac loading of the micellar solution developed in the previous experiments, in addition to the viscosifying agents, combinations of permeation enhancers were added, with the final aim of increasing API permeation through the skin. Thus, 113 semisolid formulations comprising 2.15% (w/w) diclofenac sodium were developed according to the specific compositions compiled quantitatively in Table 4 and 5.

**Table 4: Composition of the tested formulations comprising a gelling agent and permeation enhancers.**

| **Samples** | | | **Permeation enhancers (%)** | | | **gelling agent** | | **[Surfactant (%)** | |
|---|---|---|---|---|---|---|---|---|---|
| | **PPG** | **AO** | **ISO** | **C3** | **TRPDMSONMP AZO** | **XAN** | **HEC** | **Span 80** | **Tween 20** |
| A1 | 5 | 0.75 | 11.75 | 2.50 | | 1.8 | | 0.81 | 0.39 |
| A2 | 5 | 0.75 | 11.75 | 2.50 | | 1.8 | | 1.08 | 0.52 |
| A7 | 5 | 0.75 | 11.75 | 1.02 | | 1.8 | | | 0.48 |
| A8 | 5 | 0.75 | 11.75 | 1.34 | | 1.8 | | | 0.66 |
| A9 | 5 | 0.75 | 11.75 | 1.68 | | 1.8 | | | 0.81 |
| A10 | 5 | 1.20 | 11.75 | 1.02 | | 1.8 | | | 0.48 |
| A11 | 5 | 1.20 | 11.75 | 1.34 | | 1.8 | | | 0.66 |
| A12 | 5 | 1.20 | 11.75 | 1.68 | | 1.8 | | | 0.81 |
| B7 | 10 | 0.75 | | | 5 | 2 | | 0.82 | 0.68 |
| B8 | 10 | 0.75 | | | 5 | 2 | | 1.08 | 0.91 |
| B9 | 10 | 0.75 | | | 5 | 2 | | 1.35 | 1.15 |
| B10 | 10 | 1.20 | | | 5 | 2 | | 0.82 | 0.68 |
| B11 | 10 | 1.20 | | | 5 | 2 | | 1.08 | 0.91 |
| B12 | 10 | 1.20 | | | 5 | 2 | | 1.35 | 1.15 |
| C1 | 10 | | 15 | | 1.00 | | 1.6 | | |
| C2 | 10 | | 15 | | 0.75 | | 1.6 | | |
| C3 | 10 | | 15 | | 0.50 | | 1.6 | | |
| C4 | 10 | | 10 | | 1.00 | | 1.6 | | |
| C5 | 10 | | 10 | | 0.75 | | 1.6 | | |
| C6 | 10 | | 10 | | 0.50 | | 1.6 | | |
| C7 | 5 | | 15 | | 1.00 | | 1.6 | | |
| C8 | 5 | | 15 | | 0.75 | | 1.6 | | |
| C9 | 5 | | 15 | | 0.50 | | 1.6 | | |
| C10 | 15 | | 10 | | 1.00 | | 1.6 | | |
| C11 | 10 | | 15 | | 0.75 | | 1.6 | | |
| C12 | 10 | | 15 | | 0.50 | | 1.6 | | |
| C13 | 10 | | 10 | | 1.00 | 1.6 | | 0.82 | 0.68 |
| C14 | 10 | | 10 | | 1.00 | 1.6 | | 1.08 | 0.91 |
| C15 | 10 | | 10 | | 1.00 | 1.6 | | 1.35 | 1.15 |
| C16 | 10 | | 10 | | 0.75 | 1.6 | | 0.82 | 0.68 |
| C17 | 10 | | 10 | | 0.75 | 1.6 | | 1.08 | 0.91 |
| C18 | 10 | | 10 | | 0.75 | 1.6 | | 1.35 | 1.15 |
| C19 | 5 | | 10 | | 1.00 | 1.6 | | 0.82 | 0.68 |
| C20 | 5 | | 10 | | 1.00 | 1.6 | | 1.08 | 0.91 |
| C21 | 5 | | 10 | | 1.00 | 1.6 | | 1.35 | 1.15 |
| C22 | 5 | | 10 | | 0.75 | 1.6 | | 0.82 | 0.68 |
| C23 | 5 | | 10 | | 0.75 | 1.6 | | 1.08 | 0.91 |
| C24 | 5 | | 10 | | 0.75 | 1.6 | | 1.35 | 1.15 |

**Table 5: Continuation of Table 4: Composition of tested formulations comprising a gelling agent and permeation enhancers.**

| **H16 4.3% (50%) + WATER** | | | | | | | | | | | | |
|---|---|---|---|---|---|---|---|---|---|---|---|---|
| **Samples** | **Permeation enhancers (%)** | | | | | | | | **gelling agent** | | **Surfactant (%)** | |
| | **PPG** | **AO** | **ISO** | **C3** | **TRP** | **DMSO** | **HMP** | **AZO** | **XAM** | **HEC** | **Span 80** | **Tween** |
| | | | | | | | | | | | | **20** |
| E1 | 10 | | | | | 10 | | | | 2 | | |
| E2 | 20 | | | | | 5 | | | | 2 | | |
| E3 | 25 | | | | | 5 | | | | 2 | | |
| E5 | 20 | | 5 | | | 5 | | | | 2 | | |
| E6 | 25 | | 5 | | | 5 | | | | 2 | | |
| E11 | 10 | | 10 | | | 5 | | | | 2 | | |
| F1 | | | | | 13 | | 5 | | | 2 | | |
| F2 | | | | | 13 | | 8 | | | 2 | | |
| F3 | | | | | 10 | | 8 | | | 2 | | |
| F7 | 10 | | | | | | 8 | | | 2 | | |
| F8 | 20 | | | | | | 5 | | | 2 | | |
| F9 | 25 | | | | 10 | | 5 | | | 2 | | |
| F10 | 10 | | | | 5 | | 5 | | | 2 | | |
| F11 | 10 | | | | 10 | | 5 | | | 2 | | |
| F12 | 15 | | | | 5 | | 5 | | | 2 | | |
| G4 | 10 | 0.75 | 10 | | | | | 1.00 | | 2 | | |
| G5 | 10 | 0.75 | 10 | | | | | 0.75 | | 2 | | |
| G6 | 10 | 0.75 | 10 | | | | | 0.50 | | 2 | | |
| G7 | 5 | 0.75 | 15 | | | | | 1.00 | | 2 | | |
| G8 | 5 | 0.75 | 15 | | | | | 0.75 | | 2 | | |
| G9 | 5 | 0.75 | 15 | | | | | 0.50 | | 2 | | |
| G10 | 15 | 0.75 | 10 | | | | | 1.00 | | 2 | | |
| H1 | | 0.75 | 10 | | 5 | | | | 2 | | 0.82 | 0.68 |
| H2 | | 0.75 | 10 | | 5 | | | | 2 | | 1.08 | 0.91 |
| H3 | | 0.75 | 10 | | 5 | | | | 2 | | 1.35 | 1.15 |
| H4 | | 0.75 | 10 | | 10 | | | | 2 | | 0.82 | 0.68 |
| H5 | | 0.75 | 10 | | 10 | | | | 2 | | 1.08 | 0.91 |
| H6 | | 0.75 | 10 | | | | | | 2 | | 1.35 | 1.15 |
| H7 | | 0.75 | 5 | | | | | | 2 | | 0.82 | 0.68 |
| H8 | | 0.75 | 5 | | | | | | 2 | | 1.08 | 0.91 |
| I1 | 5 | 0.75 | 10 | | | | 5 | | 2 | | 0.82 | 0.68 |
| I2 | 5 | 0.75 | 10 | | | | 5 | | 2 | | 1.08 | 0.91 |
| I3 | 5 | 0.75 | 10 | | | | 5 | | 2 | | 1.35 | 1.15 |
| I4 | | 0.75 | 10 | | | | 5 | | 2 | | 0.82 | 0.68 |
| I5 | | 0.75 | 10 | | | | 5 | | 2 | | 1.08 | 0.91 |
| I8 | | 0.75 | 10 | | | | 5 | | 2 | | 1.35 | 1.15 |
| I7 | 10 | 0.75 | 10 | | | | 5 | | 2 | | 0.82 | 0.68 |
| I8 | 10 | 0.75 | 10 | | | | 5 | | 2 | | 1.08 | 0.91 |
| I9 | 10 | 0.75 | 10 | | | | 5 | | 2 | | 1.35 | 1.15 |
| I10 | 10 | 0.75 | 5 | | | | 5 | | 2 | | 0.82 | 0.68 |
| I11 | 10 | 0.75 | 5 | | | | 5 | | 2 | | 1.08 | 0.91 |
| I12 | 10 | 0.75 | 5 | | | | 5 | | 2 | | 1.35 | 1.15 |

| | | | | | | | | | | | | |
|---|---|---|---|---|---|---|---|---|---|---|---|---|
| PPG: Propylene glycol, AO: Oleyl alcohol, ISO: isopropanol, C3: Kollicream 3C, TRP: Transcutol, DMSO: dimethyl sulfoxide, NMP: N-Methyl-2-pyrrolidone, AZO: Azone or N-dodecyl caprolactam, XAN: xanthan gum, HEC: hydroxy ethyl cellulose; | | | | | | | | | | | | |

These formulations were placed in a stability chamber according to ICH conditions at 25°C/ 65% RH and 40°C/ 75%RH). After 30 days, 78 and 19 formulations remained visually stable at 25°C/ 65% RH and 40°C/ 75%RH conditions, respectively, without any evidence of aggregation/precipitation or phase separation. The 19 formulations which were stable at both conditions were conveniently characterized, and five of them resulted to be perfectly stable after 30 days.

The specific composition and characteristics of these five formulations (A7, B7, C22, H3 and I1), evaluated in three independent batches, are given in Table 6. Illustrative photographs of formulations appearance are shown in FIG. 3.

**Table 6**

| **Example A7** | |
|---|---|
| **Ingredient** | **Amount [%w/w]** |
| Diclofenac sodium | 2.15 |
| Capryol 90 | 2.5 |
| Kolliphor HS15 | 5 |
| Tween 80 | 2.5 |
| Transcutol | 2.5 |
| Oleyl alcohol | 0.75 |
| Isopropanol | 11.75 |
| Kollicream 3C | 1 |
| Tween 20 | 0.48 |
| Xantural | 1.8 |
| Purified water | q.s. ad 100 |

| **Example B7** | |
|---|---|
| **Ingredient** | **Amount [%w/w]** |
| Diclofenac sodium | 2.15 |
| Capryol 90 | 2.5 |
| Kolliphor HS15 | 5 |
| Tween 80 | 2.5 |
| Transcutol | 2.5 |
| Propylene glycol | 10 |
| Oleyl alcohol | 0,75 |
| N-Methyl-2-pyrrolidone | 5 |
| Span 80 | 0.82 |
| Tween 20 | 0.68 |
| Xantural | 2 |
| Purified water | q.s. ad 100 |

| **Example C22** | |
|---|---|
| **Ingredient** | **Amount [%w/w]** |
| Diclofenac sodium | 2.15 |
| Capryol 90 | 2.5 |
| Kolliphor HS15 | 5 |
| Tween 80 | 2.5 |
| Transcutol | 2.5 |
| Propylene glycol | 5 |
| Isopropanol | 10 |
| AZO | 0.75 |
| Span 80 | 0.82 |
| Tween 20 | 0.68 |
| Xantural | 1.6 |
| Purified water | q.s. ad 100 |

| **Example H3** | |
|---|---|
| **Ingredient** | **Amount [%w/w]** |
| Diclofenac sodium | 2.15 |
| Capryol 90 | 2.5 |
| Kolliphor HS15 | 5 |
| Tween 80 | 2.5 |
| Transcutol | 7.5 |
| Oleyl alcohol | 0.75 |
| Isopropanol | 10 |
| Span 80 | 1.35 |
| Tween 20 | 1.15 |
| Xantural | 2 |
| Purified water | q.s. ad 100 |

| **Example I1** | |
|---|---|
| **Ingredient** | **Amount [%w/w]** |
| Diclofenac sodium | 2.15 |
| Capryol 90 | 2.5 |
| Kolliphor HS15 | 5 |
| Tween 80 | 2.5 |
| Transcutol | 2.5 |
| Isopropanol | 10 |
| Propylene glycol | 5 |
| Oleyl alcohol | 0.75 |
| N-Methyl-2-pyrrolidone | 5 |
| Span 80 | 0.82 |
| Tween 20 | 0.68 |
| Xantural | 2 |
| Purified water | q.s. ad 100 |

**Table 7: Characterization a one month ICH stability of selected micellar-based gel-like formulations containing a final 2.15 % (w/w)diclofenac sodium loading. [Drug Up/down % means drug content, compared to initial drug content in the samples from the upper (Up) and lower (down) half of the storing-vials]**

| | Initial characterization | | | Time (months)= 1 | | | | | | | |
|---|---|---|---|---|---|---|---|---|---|---|---|
| | | | | 25°C ± 2°C/60% RH ± 5% | | | | 40°C ± 2°C/75% RH± 5% | | | |
| USC Code | pH | Size (nm) | Drug (%) | pH | Size (nm) | Drug Up (%) | Drug Down (%) | pH | Size (nm) | Drug Up (%) | Drug Down (%) |
| A7 | 7.4 | 54±11 | 98.3±2.3 | 7.4 | 31±8 | 96.67+3.5 | 96.7±2.9 | 7.3 | 46±21 | 101.3±1.4 | 101.32 |
| B7 | 7.3 | 43±10 | 97.9±5.7 | 7.3 | 44±12 | 91.6±4.1 | 92.4±4.6 | 7.1 | 38±7 | 42.15±10.4 | 55.13±30 |
| C22 | 7.5 | 44±4.8 | 99.1±3.7 | 7.5 | 33±10 | 95.9±1.5 | 106.4±1.1 | 7.4 | 37±7 | 102.1±3.1 | 102.6±3.6 |
| H3 | 7.4 | 34±8.7 | 101.1±2.5 | 7.5 | 26±3 | 99.8±2.6 | 103.8±2.5 | 7.4 | 31±6 | 106.0±1.1 | 102.7±2.3 |
| I1 | 7.4 | 49±2.5 | 98.4±3.1 | 7.4 | 34±9 | 98.7±3.7 | 98.7±2.3 | 7.4 | 35±6 | 103.3±1.7 | 98.5±3.7 |

| | | | | | | | | | | | |
|---|---|---|---|---|---|---|---|---|---|---|---|
| * Probable analytical/sampling error | | | | | | | | | | | |

### Embodiments

Embodiments described herein can be understood more readily by reference to the detailed description, examples, and figures. Elements and methods described herein, however, are not limited to the specific embodiments presented in the detailed description, examples, and figures. It should be recognized that the exemplary embodiments herein are merely illustrative of the principles of the present invention. Numerous modifications and adaptations will be readily apparent to those of skill in the art without departing from the spirit and scope of the invention.

In addition, all ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" or "5 to 10" or "5-10" should generally be considered to include the end points 5 and 10.

It is further to be understood that the feature or features of one embodiment may generally be applied to other embodiments, even though not specifically described or illustrated in such other embodiments, unless expressly prohibited by this disclosure or the nature of the relevant embodiments. Likewise, compositions and methods described herein can include any combination of features and/or steps described herein not inconsistent with the objectives of the present disclosure. Numerous modifications and/or adaptations of the compositions and methods described herein will be readily apparent to those skilled in the art without departing from the present subject matter.

## Claims

1. A micellar solution for topical drug delivery, comprising:
**a)** diclofenac alkali metal salt, in a concentration of about 2% to about 5.5% (w/w),
**b)** diethylene glycol monoethyl ether, in a concentration of about 3% to about 7% (w/w),
**c)** propylene glycol esters of C₆ to C₁₂ fatty acids,
**d)** polyethylene glycol esters of C₁₄ to C₁₈ fatty acids,
**e)** polyethylene glycol esters of sorbitan C₁₆ to C₂₀ fatty acid monoester, and
**f)** water,
wherein c) and d) together are present in a concentration of about 10% to about 25% (w/w), and
wherein e) is present in a concentration of about 5% to about 10% (w/w).

2. A micellar solution according to claim 1, wherein c) has a hydrophilic lipophilic balance value of about 5 to about 7.

3. A micellar solution according to claim 1 or 2, wherein d) has a hydrophilic lipophilic balance value of about 15 to about 17.

4. A micellar solution according to claim 1, 2 or 3, wherein e) has a hydrophilic lipophilic value balance of about 14 to about 16.

5. A micellar solution according to any of the preceding claims, wherein c) is present in a concentration of about 5% to about 15% (w/w).

6. A micellar solution according to any of the preceding claims, wherein c) is propylene glycol monocaprylate.

7. A micellar solution according to any of the preceding claims, wherein d) is present in a concentration of about 10% to about 20% (w/w).

8. A micellar solution according to any of the preceding claims, wherein d) is a mixture of polyethylene glycol mono- and diesters of 12-hydroxystearic acid comprising about 20% to about 40% (w/w) free polyethylene glycol.

9. A micellar solution according to any of the preceding claims, wherein e) is polyethylene glycol sorbitan monooleate.

10. A micellar solution according to any of the preceding claims, wherein the micellar solution is transparent.

11. A micellar solution according to any of the preceding claims, having an average micelle size, determined by dynamic light scattering, in the range of about 5nm to about 10nm.

12. A micellar solution according to any of the preceding claims, wherein the micelles of the micellar solution form spontaneously.

13. A micellar solution according to any of the preceding claims, comprising:
a) about 4% to about 5% (w/w) diclofenac sodium,
b) about 5% (w/w) diethylene glycol monoethyl ether,
c) about 5% (w/w) Capryol 90,
d) about 10% (w/w) Kolliphor HS15,
e) about 5% (w/w) Tween 80, and
f) water.

14. A micellar solution according to any of the preceding claims, for use in the treatment of joint pain, osteoarthritis, muscle pain, back pain and/or inflammation.

15. A micellar solution according to any of the preceding claims, provided in a container, the container comprising an applicator for direct application onto the skin.

16. A gel for topical drug delivery, comprising
**a)** the micellar solution according to any of the preceding claims, in a concentration of about 40% to about 80%,
**b)** a gelling agent, selected from xanthan gum, hydroxyethyl cellulose, and any combination thereof.

17. A gel according to claim 15, further comprising
**c)** a permeation enhancer selected from C₁₂ to C₂₀-fatty alcohol, esters of C₈ to C₁₀-saturated fatty acids with C₁₂ to C₁₈-fatty alcohols, N-methyl-2-pyrrolidone and N-dodecyl caprolactam.

18. A gel according to claim 16 or 17, further comprising:
**d)** a C₂ to C₅ poly alcohol,
**e)** isopropanol, in a concentration of about 8% to about 12% (w/w),
**f)** sorbitan monooleate, and
**g)** polysorbate 20.

19. A gel according to claim 14, wherein
**b)** is xanthan gum, present in a concentration of about 1.5% to about 2.5% (w/w), and
**c)** is oleyl alcohol, present in a concentration of about 0.75% (w/w).
